# EUROPEAN PATENT APPLICATION

(11) **EP 2 050 479 A2**
(43) Date of publication of application: **22.04.2009**
(21) Application number: 08396007.0
(22) Date of filing: 20.05.2008
(51) Int. Cl.: A61M 16/18

(54) **Apparatus, system and method for admistering an anesthetic agent for a subject breathing**

(30) Priority: 16.10.2007 EP 07396006; 31.01.2008 EP 08396003
(71) Applicant: GENERAL ELECTRIC COMPANY, Schenectady, NY 12345 (US)
(72) Inventor: Haveri, Heikki, 03150 Huhmari (FI); Heinonen, Erkki, 00750 Helsinki (FI)
(74) Representative: Kanerva, Arto

(57) **Abstract**

An apparatus for administering an anesthetic agent for a subject breathing is disclosed herein. The apparatus includes a liquid reservoir (9) for an anesthetic agent and an anesthetic agent dosing unit (7) in fluid connection with the liquid reservoir (9) and which anesthetic agent dosing unit is connectable to an airway tube for delivering breathing gases to a subject, the dosing unit including a first member (27) to produce anesthetic agent droplets having a diameter less than 100 µm.

## Description

### BACKGROUND OF THE INVENTION

This disclosure relates to an apparatus, a system and a method for administering an anesthetic agent for a subject breathing.

While anestetizing patients anesthetic agent is typically held in a vessel having a liquid space and a gas space in which vessel the agent is vaporized into a carrying gas, which may require heating depending on physical features of the agent. The vaporized anesthetic agent mixed with the carrying gas is then led to the patient for inspiration. However, vaporizers like these are producing a vapor continuously and not at times when the vapor is useful to feed to the patient. Also it takes a lot of room, which is limited in operating rooms where these devices are used.

Unnecessary to say the described inhalation anesthesia delivery system is complex and requires a lot of infrastructure. This is one aspect promoting an intravenous anesthesia where a drug is infused as a bolus or continuous flow directly to a patient's vein. However, even then, a possibility for an inhalation anesthesia delivery is often preserved as a backup for the intravenous injection and for the patients developing some allergic reaction to the intravenous drugs. Therefore, the inhalation system complexity is present even in intravenous anesthesia. The inhalation anesthesia is increasingly used also in an intensive care departments to sedate the patients. However, the ICU departments lack totally the required system infrastructure and often also a room for that. Other places where the inhalation anesthesia demand exists but infrastructure is often missing are all kind of field conditions and developing countries. Also commercially available for limited inhalation agents are the inhalation anesthesia delivery systems having the anesthesia agent preserving capability comparable with a state of the art anesthesia practice. This can be provided using an agent reflector connected to an airway tube in the reciprocating gas flow path between the patient and a circuit Y-piece. There the reflector adsorbs agent from an exhalation gas and releases to an inspiration gas. Such provide agent preserve capacity respective to 1-1.5 L/min fresh gas flow of the re-breathing system. Using the reflector removes the need for a CO2 adsorber when the expired gas is not circulated back to the inspiration. Furthermore, also a fresh gas mixer is eliminated, as ventilator when controlling the circuit pressure provides the breathing gas.

When the reflector prevents the anesthesia agent flow-through, the agent needs to be delivered into or between the reflector and the patient. The agent may be administered e.g. as a liquid infusion, as gasified on an anesthetic vaporizer, as aerosol droplets produced with a nebulizer, or as a bolus source embedded in the system that can be activated to release the agent. The liquid infusion and the nebulizer has been used to deliver the anesthetic agent.

The agent injection carried out with a micro pump or injections of the highly pressurized agent through a nozzle are well-known techniques to form small droplets. However, one of the fundamental problems in both of these techniques is how to produce an adequate number of droplets per time unit, or in other word how to vaporize an adequate volume of the anesthetic per time unit, to reach high enough concentration of the anesthetic agent in the breathing gas of a patient, in other words an adequate level of the anesthesia.

Injecting the highly pressurized agent through a nozzle is a simple technique to produce droplets of anesthetic agent, but the drop size distribution of produced droplets is wide. Smallest droplets vaporize fast, but larger droplets may enter the patient's respiratory system as the liquid causing harm to the patient's respiratory system. Decreasing the nozzle size can decrease the drop diameter but when the drop diameter is deliberately decreased the drop volume also decreases and the total volume of the anesthetic agent delivered in time unit also decreases fast. The number of drops and thus the total volume of the anesthetic agent delivered can be increased by increasing the applied pressure to a certain extent. However, increasing the pressure causes risk for the patient as the high pressure may damage the lungs due to the miss-operation of the device. Of course the number of nozzles can be increased also, which makes the device even more complex, heavier and clumsier.

The agent injection carried out with the micro pump is more sophisticated technique to produce even diameter droplets of the anesthetic agent furthermore the droplet diameter can be controlled better. On the other hand, because of the limits of the available technology, the minimum droplet diameter is normally more than 20 µm, usually at the range of 100 µm, causing a real risk of harm for the patient, as droplets may enter the patient's respiratory system as the liquid.

The technology is widely used in ink jet printers. Currently the micro pump, having a piezo-electric vibrator connected to a piston, can contain up 128 pump heads with an overall size about 1 cubic inch. Also larger micro pumps containing more than 5000 pump heads can be made, but the width of such element is more than 11 inches. Such micro pump is not suitable for agent delivery use as the overall size of whole device would be much too big. In the micro pump the piston moves longitudinally inside the channel, where the pumped liquid is also applied through a perpendicular cannel in connection with the longitudinal channel. Each longitudinal back and forth movement pumps a drop of the liquid through the nozzle adjacent to the piston. The size and the output diameter of the nozzle mainly determine the drop diameter, but it can be slightly altered with the movement of the piston. To get certain size of droplets the nozzle diameter must be much smaller than the diameter of the produced droplet. A viscosity of the liquid and the flow resistance of the nozzle and cavities in connection with the nozzle and the piston limit the frequency of the pumping action of the liquid also called a nozzle frequency. The nozzle frequency is less than 20 kHz with the technology available at the moment.

The drop diameter is limited by the diameter of the nozzle. Smaller nozzles are difficult to produce and also with a reasonable cost. The diameter of the smallest commercially available nozzles is approximately 10-20 µm and they can produce droplets having the diameter of approximately 20-100 µm. Furthermore the number of the pump heads in the micro pump that can be produced limits the quantity of the drops that can be produced. More pump heads are certainly needed, as the drop diameter is decreased, to produce large enough volume of the anesthetic agent in the time unit to sedate the patient. The pump head containing 128 nozzles in which the diameter of each nozzle is 10 µm and the nozzle frequency is 20 kHz, the total volume of the anesthetic that can be transformed in to small droplets having diameter of 20 µm within one second is approximately 0.006 ml. Such volume of the anesthetic, which is about the maximum volume of the anesthetic liquid that can be produced with the micro pump implemented with currently available technology in an acceptable device size, is much too low for sedating the patient and even reaching sufficient level of the anesthesia in a steady state. Increasing the number of the pump heads in the micro pump also increases the device size, the power consumption and the device complexity making the device improper. Increasing the droplet size causes a real risk of harm for the patient, as droplets may enter the patient's respiratory system as liquid.

In the techniques described above, to overcome the disadvantage of producing too large droplets, a heater element can be placed inside the breathing circuit between the delivery device and the patient. This is to ensure that too large droplets evaporate faster and do not enter the patient as the liquid. The disadvantage in this is that the heater element causes a risk of a leakage current as well as the heater element may brake in to pieces and enter the patient's lungs.

### BRIEF DESCRIPTION OF THE INVENTION

The above-mentioned shortcomings, disadvantages and problems are addressed herein which will be understood by reading and understanding the following specification. In an embodiment, an apparatus for administering an anesthetic agent for a subject breathing includes a liquid reservoir for an anesthetic agent and an anesthetic agent dosing unit in fluid connection with the liquid reservoir and which anesthetic agent dosing unit is connectable to an airway tube for delivering breathing gases to a subject, the dosing unit including a first member to produce anesthetic agent droplets having a diameter less than 100 µm.

In another embodiment, a system for administering an anesthetic agent for a subject breathing includes an anesthetic agent dosing unit, a gas analyzing apparatus and a user interface. The system for administering an anesthetic agent also includes an anesthetic agent delivery controller connected to the gas analyzing apparatus for receiving measurement results and also connected to the user interface for receiving a target anesthetic agent concentration and further connected to the anesthetic agent dosing unit for controlling an anesthetic agent delivery based on received measurement results and the target anesthetic agent concentration. The anesthetic agent dosing unit is adapted to deliver anesthetic agent droplets having a diameter less than 100 µm.

In yet another embodiment a method for administering an anesthetic agent for a subject breathing includes delivering a breathing air including at least oxygen for the subject breathing, delivering a liquid containing the anesthetic agent and breaking up the liquid into droplets having a diameter less than 100 µm. The method for administering an anesthetic agent also includes delivering the droplets into the breathing air, evaporating the droplets into the breathing air and supplying the evaporated anesthetic agent mixed with the breathing air for the subject breathing.

Various other features, objects, and advantages of the invention will be made apparent to those skilled in art from the accompanying drawings and detailed description thereof.

### BRIEF DESCRIPTION OF THE DRAWINGS

Figure 1 is a general view of a system in accordance with an embodiment;

Figure 2 is a schematic view of a delivery device of the embodiment of the invention; and

Figure 3 is a schematic view showing an operation of the delivery device of Figure 2.

### DETAILED DESCRIPTION OF THE INVENTION

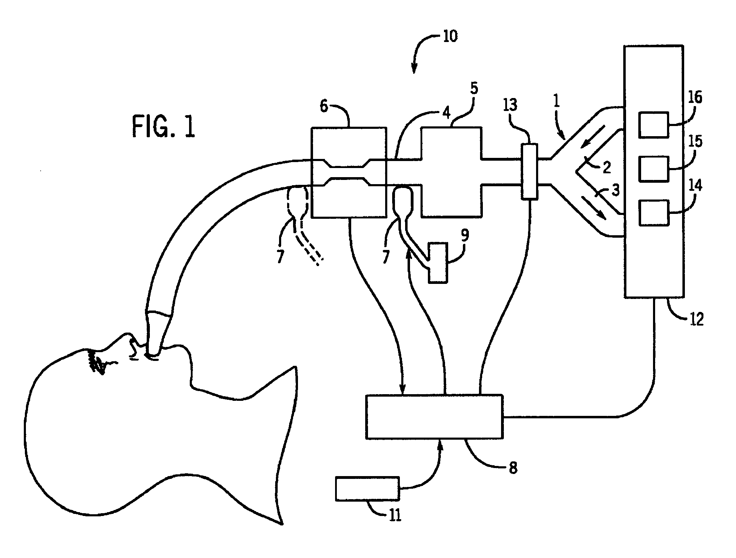

Figure 1 shows an anesthesia delivery system 10 comprising a breathing connector 1, such as Y-piece, having three branches. The first branch is an inhalation tube 2 for delivery of inhaled gases during an inspiration, the second branch is an exhalation tube 3 carrying expired gas during an expiration and the third branch is an airway tube 4 carrying during the inspiration the inhaled gas to and during the expiration the expired gas from a subject. Also the anesthesia delivery system 10 may comprise an anesthetic agent adsorber unit 5, such as a reflector, for adsorbing an anesthetic agent usually when a subject is exhaling and for releasing this agent into inspiratory breathing air. Further the anesthesia delivery system 10 comprises a gas analyzing apparatus 6 and an anesthetic agent dosing unit 7, which both are assembled into the airway tube 4. The gas analyzing apparatus 6, such as an infrared gas analyzer, is for measuring a concentration of at least one component of a gas flowing along the airway tube 4. The anesthetic agent dosing unit 7, such as a nebulizer, is for dosing anesthetic agent into the breathing air flowing along the airway tube 4. The anesthesia delivery system also comprises an anesthetic agent delivery controller 8 and a user interface 11. The anesthetic agent delivery controller 8 is adapted to control a liquid delivery into the breathing air and to control the liquid delivery from a liquid reservoir 9 to the anesthetic agent dosing unit 7. Further the anesthetic agent delivery controller 8 is adapted to receive measurement results from the gas analyzing apparatus and also to receive a target anesthetic agent concentration from a user interface 11.

In Figure 1 a breathing circuit represents an open one. For a control of the subject's breathing there is a ventilator 12 in a flow connection with the inhalation tube 2 and the exhalation tube 3 of the breathing connector 1. The ventilator 12 is to deliver the breathing air including at least oxygen for the subject breathing. The ventilator 12 comprises an inspiration controller 14 that regulates the breathing gas mixture and flow rate for the breathing gas delivery to the subject, and an expiration controller 15 regulating the subject's expiration flow and pressure. The ventilator further comprises a timing unit 16 to control a duration of the inspiration and expiration.

The liquid delivery by means of the anesthetic dosing unit 7 may happen by transforming or breaking up the liquid into small droplets or "spray" that is immediately evaporated into the breathing air such as the inspiratory gas flow. Because of their small size, large surface area, and high vapor pressure of the volatile anesthesia agents, these are evaporated into the breathing gas rapidly and spontaneously before supplying the evaporated anesthetic agent mixed with the breathing air for the subject breathing.

The target anesthetic agent concentration fed to the user interface 11 may be a single value or a range having minimum and maximum values. The anesthetic agent delivery controller 8 is adapted to adjust the anesthetic agent delivery into the breathing air flowing along the airway tube 4 based on the measurement results from the gas analyzing apparatus 6 and the target anesthetic agent concentration in order to match the measurement result with the target anesthetic agent concentration. The anesthetic agent delivery controller 8 receives a power e.g. from a separate power source not shown in the figure, or from the ventilator 12.

Preserving the anesthetic agent in the subject's breathing air effectively, the decrease of the gas concentration after stopping the delivery is slow. To make the control easier, the user given target concentration may also have a time forecast when the anesthesia is likely to stop. Using this information the system can begin preparation in reducing the delivery to obtain the given target at a given time.

The anesthetic agent dosing unit 7 may be connected upstream (solid line) or downstream (dashed illustration) of the gas analyzing apparatus 6, but the anesthetic agent dosing unit 7 is preferably downstream of the breathing connector 1 towards the patient. This is to minimize the volume of evaporated anesthetics in the breathing circuit gas circulation not important for sedating the patient thus to minimize the agent wasted. The delivery may also be synchronized with the patient breathing to deliver either during the inspiration or the expiration. For this purpose the anesthesia delivery system may be equipped also with a flow phase detector 13, such as a flow sensor, to detect the subject flow phase or this same information can be acquired from the ventilator 12 including the flow phase detector 13 to detect the subject flow phase.

Further, if the gas analyzing apparatus 6 is fitted to measure also a patient breathing gas CO2 concentration, which can be applied on the flow phase detection purpose as well. In this case the expiration phase is identified when the measured CO2 concentration exceeds a given CO2 value and respectively during the inspiration is below the given CO2 value. A delivery synchronization corresponds with the delivery point location advantageously so that the aerosol delivered does not end up to the measurement point immediately upon delivery to avoid a disturbing uninformative measurement noise caused by high-concentration delivery doses. The upstream connection would then correspond to the synchronization with the expiration phase and would provide the longest time for the vaporization. The downstream delivery would then be synchronized with the inspiration phase respectively. In the former case the delivery dose would first be adsorbed in the anesthetic agent adsorber unit 5 and then released to the coming inspiration flow. In the latter case the dose becomes diluted in the alveolar volume of the subject.

A result of the system described is very small and compact system when each of the anesthetic agent dosing unit 7, the anesthetic agent adsorber unit 5 and said gas analyzing apparatus 6 is mounted on the airway tube 4. All extra wires or tubes connecting the system to any outside device are avoided making a working environment around the patient more safe from both the patient's and the personnel's point of view. The anesthetic agent dosing unit 7 has to be also electrically low powered and electrically isolated from the patient to avoid any electrical hazards for the patient and for the user.

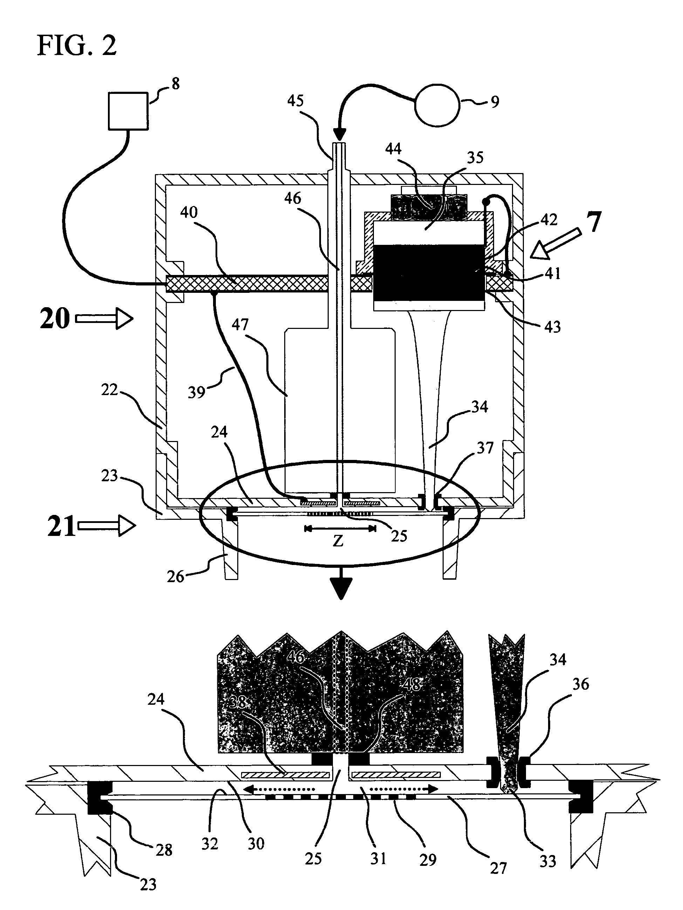

Figure 2 shows an embodiment of the anesthetic agent dosing unit 7 comprising a device body 20 and a patient connector 21, which connect together. The anesthetic agent delivery controller 8 containing electronics, software and electrical power source to control the device, may be located further, but as well it can be integrated inside the anesthetic agent dosing unit 7. The liquid form anesthetic agent is stored into the liquid reservoir 9, which can be located further away or close to or within the anesthetic agent dosing unit 7, from where the liquid is delivered in to the anesthetic agent dosing unit 7. The anesthetic agent dosing unit 7 is further connected to the breathing connector 1 or especially to the airway tube 4 by means of the patient connector 21.

Volatile anesthetic fluids are very difficult to handle as they are powerful solvents, their surface tension is very small and they tend to pass through even the smallest openings. Therefore parts that are in straight contact with the anesthetic should be made of materials such as an anesthetic endurable metal, but preferably an anesthetic endurable plastic or similar to make the device less expensive. Beneath a schematic view of the anesthetic agent dosing unit 7 of Figure 2 there is a more detailed, enlarged view of the functional part that breaks up the liquid into small droplets or spray. The device body 20, which comprises a first housing 22 is reusable and thus non-disposable and therefore all the technically complicated, more expensive components are placed inside it. The patient connector 21 is designed detachable and disposable and therefore it has to be very simple and low-cost, but of course it can be cleanable and reusable as well. The benefit of the disposability is due to the fact that the patient connector 21 separates the patient side from the device side preventing bacteria and viruses to enter the reusable parts. This reduces the cleaning work done by the hospital personnel and improves the patient safety.

Patient connector 21 comprises a second housing 23 preferably made of the anesthetic endurable plastic or similar. The second housing 23 has a bayonet type of connection on its surfaces towards the first housing 22, having a wall 24 with an aperture 25 for the liquid, or similar to connect patient connector 21 to the first housing 22 of the device body 20 and a second connector 26 which may be of luer fitting type or similar to connect the patient connector 21 to the airway tube 4. A first member 27 is fitted and sealed in to the second housing 23 through a first sealing 28, which is advantageously made of the anesthetic endurable elastic material, to prevent the liquid anesthetic to traverse or leak to the patient side of the patient connector 21, but it is also used to separate the patient side of the breathing circuit from the side of the anesthetic agent dosing unit 7 to prevent a contamination between different patients.

The first member 27 such as a mesh plate has a group of holes 29, for example machined with laser, chemically etched or similar, in to the area in the middle of first member 27. A diameter of straight holes, which can also be conically or similarly shaped, is more than 100 nm, but less than 100 µm and thus preferably between 100 nm-100 µm and a height of holes 29 becomes from a thickness of first member 27, which is preferably between 10-100 µm. The first member 27 can be made of any material suitable for establishing vibrations at ultrasonic frequencies, but preferably it is made of an electrically conductive metal such as a stainless steel, brass or similar. The first member 27 can also be implemented by gluing, soldering or similarly attaching a smaller diaphragm, made of a metal, ceramic or similar material, having the holes 29 in to an opening in the larger first member made of the metal or similar that function as a rigid frame or a body for the diaphragm to compose the first member 27.

When the patient connector 21 is connected to the device body 20, the first sealing 28 in the patient connector 21 tightens against a lower surface 30 of the device body 20 forming a chamber 31, which is advantageously sealed, between a lower surface 30 of the device body 20 and an upper surface 32 of the first member 27.

The first member 27 settles close to the lower surface 30 of the device body 20 so that the upper surface 32 of first member 27 touches a tip 33 of a transmitter 34 of a vibrator 35, which perforates the wall 13 of the first housing 22 and goes through a second sealing 36, which may be made of the anesthetic endurable elastic material, which is used to prevent the anesthetic to flow inside the device body 20 and thus the first housing 22. Instead of an aperture 37 on the wall 24 there may be a wall or a part of a wall made of flexible material bending towards the vibrating first member whereby the transmitter 34, which extends towards the chamber 31, is able to vibrate the first member without piercing the wall 24.

The dielectric of the volatile anesthetics is high and the capacitance they produce between the capacitive electrodes is also high and thus the amount of the liquid can be easily measured by measuring the capacitance between an electrically conductive element 38, such as a conductive plate, inside the lower surface 30 of the wall 24 of the first housing 22 made of the plastic or similar and the electrically conductive vibrating first member 27 in the patient connector 21. The element 38 and the first member 27 thus function as the electrodes for a capacitor and the space between the element 38 and first member 27 in chamber 31 function as a dielectric, which value alters between the dielectric of an air which is the case there is no anesthetic in the chamber 31 and the maximum dielectric as the space between the element 38 and the first member 27 in the chamber 31 is filled up with the anesthetic. The capacitive measurement becomes more sensitive as the distance between the element 38 and the first member 27 is decreased whereas the amount of the anesthetic on the plate is also decreased. A suitable distance between the element 38 and the first member 27 is between 0.2 mm to 2 mm. The volatile anesthetics are good electrical insulators and thus their electrical conductivity is small so it is also possible to have electrically fully conductive lower surface 30 of the device body 20 to enable for example the capacitive measurement.

An electrical connection 39 to the element 38 can be established from an electronics board 40 in the case the device body 20 is made of the plastic, whereas the electrical connection to the vibrating first member 27 can be implemented through the tip 33 of the transmitter 34 of the vibrator 35 that also connects to the electrical ground of the anesthetic agent delivery controller 8. The vibrator 35 is preferably a piezoelectric vibrator or similar, which is controlled by the anesthetic agent delivery controller 8. The vibrating motion generated by the vibrator 35 is normally too low for a practical use and so it is necessary to magnify or amplify the vibrating motion. This function can be implemented with a horn shaped ultrasonic vibrator-transmitter 35, 34 shown in figure 2. The vibrator 35 has a construction of thin piezoelectric elements 41 preferably a shape of rings, normally two or four, that are clamped between a pair of acoustically low loss metal end masses 42, 43, preferably made of aluminum or titanium, composing a resonant element functioning in the compression mode. The assembly would be designed so that the overall length is one half-wave at the required frequency of the operation, although they can be designed in multiples of half wavelengths also. Two piezoelectric elements 41 are positioned near to the point of maximum stress in a half-wave resonant assembly. Because the elements are pre-polarized they can be so arranged that they are mechanically aiding but electrically opposing. This feature enables both end masses 42, 43 to be at a mechanical earth potential, which is in practice also the connection point of the vibrator 35 to the electronics board 40 and through that to the device body 20. The assembly is clamped together by means of a high tensile bolt 44, which ensures the ceramics are in compression at maximum vibrator displacement. The vibrators 35 constructed in this way can have potential efficiencies of 98% and will handle power transfers of hundreds of watts when employed in a mode of continuous operation. The maximum peak to peak displacements at the vibrator radiating face would be around 100-200 microns when operating at a frequency of 1-500 kHz.

The liquid form anesthetic agent is delivered from the liquid reservoir 9 to the anesthetic agent dosing unit 7 through an inlet 45 and a liquid cavity 46 of a liquid controller 47 for guiding the flow of the liquid. The liquid controller 47 for guiding the flow of liquid is preferably positioned inside the first housing 22 of the device body 20, but can be located between the inlet 45 and the liquid reservoir 9 as well. The liquid controller 47 can be controlled by the electrical signal from the anesthetic agent delivery controller 8, to apportion anesthetic along the liquid cavity 46 through a third sealing 48 and the aperture 25 opening into the chamber 31. The control of the liquid controller 47 for guiding the flow of the fluid is for instance based on the presence or the amount of liquid anesthetic in the space between the conductive element 38 and first member 27, which is known in the art. The value of the capacitance proportional to the amount of the liquid in chamber 31 is transmitted to the anesthetic agent delivery controller 8 as an electrical signal, which is then used for controlling the liquid controller 47. Thus if there is no or a little amount of the anesthetic liquid between the conductive element 38 and the first member 27, the anesthetic agent delivery controller 8 gets the electrical signal of low capacitance and the liquid controller 47 for guiding the flow of the liquid is opened to allow the flow of anesthetic liquid into the chamber 31 as long as the diameter of the fluid column between the conductive element 38 and the first member 27 exceeds the edges of the conductive element 38, which means that the capacitance reaches its maximum value and the group of the holes 29 are wetted. Then the anesthetic agent delivery controller 8 gets electrical signal proportional to maximum capacitance and the liquid controller 47 for guiding the flow of the fluid is closed again to stop the flow of the liquid until the liquid column between the conductive element 38 and the first member 27 degreases below the edges of the conductive element 38 due to spraying of the liquid and is opened again.

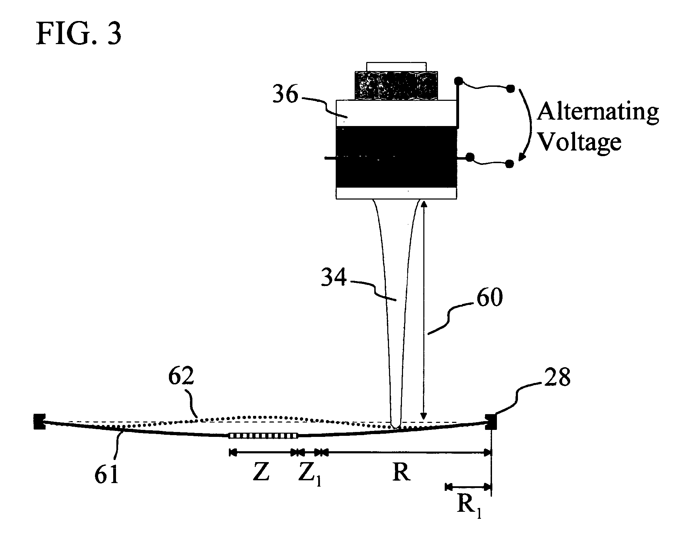

To generate the spray or anesthetic vapor an alternating electrical signal is connected to vibrator 35. The electrical energy is converted to a longitudinal mechanical movement of the transmitter 34 that is transferred via tip 33 of transmitter 34 towards the vibrating first member 27, positioned transversely against the transmitter 34, which is then forced to vibrate in the bending mode. The liquid apportioned by the liquid controller 47 for guiding the flow of the liquid on the vibrating first member 27 is squeezed through the holes in the middle of the first member 27 as little droplets as a result of back and forth movement of the first member 27, which droplets then evaporate into the inspiratory breathing air flowing in the breathing circuit. The longitudinal movement of vibrator 35 can be magnified depending on the contact point of the tip 33 and the vibrating first member 27 along the radius of the first member 27. The magnification increases proportional to the distance of the contact point measured from the center, along the radius, towards the circumference of the first member 27. Of course the magnification also depend on the applied frequency of the mechanical vibration of the vibrator 35, as well as the mechanical stiffness of the first member 27. The amplitude of the vibration may be smaller when the fundamental frequency of a longitudinal vibration 60 of the vibrator 35 is too high in regard to the mechanical stiffness of the first member 27, as shown in a simplified schematic view in figure 3. Mechanically too soft first member 27 starts to vibrate at the frequency of one of the sub-harmonics 62 of a fundamental frequency of the vibrator 35 and the amplitude of the vibration may be smaller compared to the amplitude of the vibration 61 of rigid enough first member 27 vibrating at fundamental frequency.

The tip 33 of the transmitter 34 has a contacting point on said vibrating first member 27 advantageously only in an area R and outside an area Z of the group of the holes 29, which is between an outer edge of the vibrating first member 27 and the area Z. Thus the area Z covers besides the group of the holes 29 but also the area between these holes. Furthermore it may be advantageous and even necessary to extend the area Z towards the outer edge of the vibrating first member by a distance Z₁, by leaving more space around the holes 29 in the area Z to increase the durability of the holes 29 since the vibrating tip 33 may fracture isthmuses between the holes 29 easily if the tip 33 is placed too close to the area Z of the holes 29. It is also beneficial to leave a distance R₁ between the tip 33 and the outer edge of the vibrating first member 27 although the amplification of the bowing of the vibrating first member 27 increases rapidly as the tip 33 is moved closer to the outer edge of the vibrating first member 27. If the distance R₁ is too small the transmitter 34 has to do more work against the elastic first sealing 28 to bow the vibrating first member 27, which decreases the efficiency to produce vibrations, furthermore as the vibrating first member 27 starts to bow due to the increase in length of the transmitter 34 within one cycle of the longitudinal vibration the tip 33 may slip and bend towards the center of the vibrating first member 27 braking the transmitter 34 or deteriorating its functioning. The advisable distance for Z₁ from the edge of the area Z towards the edge of the vibrating first member 27 is approximately 0.5-2 mm, whereas the advisable distance for R₁ from the edge towards the center of the vibrating first member 27 is approximately 1/6 of the radius of the first member 27. Further a contacting point might be only on a sector of said vibrating first member 27, which is less than 360 degrees around its periphery. It may well be only on the sector of the vibrating first member 27, which is less than 180 degrees around its periphery. It is quite possible to arrange the contacting point only on the sector, which is less than 90 degrees around the periphery of the first member, which is the case shown in figures 2 and 3.

The amount of the evaporated anesthetic can be regulated by adjusting the spray time and since it is optimal to spray anesthetic in to the inspiratory gas to improve the delivery efficiency, which is the actual volume of the anesthetic agent delivered in to the patient, it is useful to adjust the spray time between the start and the end of the inspiration. Alternatively the spray efficiency can be adjusted. The production rate of the evaporated agent is independent of the fresh gas flow, which reduces the consumption of the volatile anesthetics during the anesthesia. To ensure that the anesthetic agent does not seep through the holes 29 in the first member 27 while the spraying is turned of during the expiration it is useful to close the liquid controller 47 for guiding the flow of the liquid and to disable the capacitive measurement by the anesthetic agent delivery controller 8 thus preventing the liquid to flow in to the chamber 31 despite of the signal state of the capacitive measurement. As the inspiration starts again the capacitive measurement is enabled and the anesthetic agent delivery controller 8 allows the liquid controller 15 for guiding the flow of the liquid to be turned on again to feed the liquid in to the chamber 31 to keep the surface of the first member 27 wetted.

It is advantageous to deliver the agent during inspiration only to minimize the volume of the agent wasted during the expiration. Thus advantageously the device should be controllable so that the anesthetic agent can be sprayed in to the inspiratory flow, whereas the delivery can be turned off during expiration to save anesthetics. For example in an open loop controlled anesthesia, the evaporated agent delivered during the expiration does not reach the patient's lungs and increase the level of anesthesia, but traverse through the exhalation tube 3 into scavenging and is wasted. The same applies when the device is used with the anesthetic agent adsorber unit 5. The efficiency of the current anesthetic agent adsorber units are approximately 70% thus the volume of delivered anesthetics has to be approximately 1/3 higher than calculated to compensate the loss. This also means that anesthetics delivered during the expiration flow in to the anesthetic agent adsorber unit 5 and approximately 1/3 of that volume is wasted and 2/3 is reflected back to the patient. Thus it is also feasible to deliver the anesthetic agent in to the breathing circuit downstream the anesthetic agent adsorber unit 5 towards the patient, but to increase the delivery efficiency the agent should be sprayed within inspiration only.

On the other hand the delivery efficiency, or the volume of the anesthetic that can be delivered in to the patient within the time unit, should be at least sufficient enough to maintain the sedation, but preferably it is approximately 10-30 times higher, depending on the patient and the anesthetic agent used, to ensure fast sedation also during an induction. The volume of the anesthetic agent that has to be delivered within each breath to achieve the normal induction at the beginning of the sedation is between 0.1 - 1 ml. For example for Sevoflurane the typical maximum volume of the liquid anesthetic used is approximately 0.3 ml/breath during the induction, whereas the required volume of the liquid anesthetic at the steady state is typically 0.03 ml/breath only, which is about 1/10 of the maximum volume.

The surface area of sprayed, small droplets is vast and optimum diameter droplets evaporate fast in to the inspiratory gas flow before flowing in to the patient's respiratory system. The drop size or its diameter should be less than 100 µm, but preferably less than 10 µm and still better would be less than 5 µm, to ensure complete the evaporation of the anesthetic agent before entering in to the patient. On the other hand to obtain the adequate delivery efficiency during the induction also the total volume of drops produced must equal to the volume of the liquid anesthetic of at least 1 ml/breath to ensure the sedation of largest patients also. In general adjusting the drop diameter, drop the quantity or the delivery time can control the volume of the liquid agent that is sprayed and evaporated. The delivery time is limited by the duration of the inspiration and depends on the patient. The delivery time, the drop diameter and the drop quantity are difficult to adjust with conventional techniques and furthermore the drop diameter and the drop quantity are usually inversely proportional to each other.

The device shown in Figure 2 based on the vibrating mesh plate enables convenient way to produce optimum diameter droplets between 1-5 µm, with the vibrating first member 27 containing up to 10000 holes or more in such quantities of 0.1-1 ml that the total volume of the anesthetic agent delivered within each breath is enough for the induction at the beginning of the sedation also. The device with the vibrating first member 27 containing a matrix of very small holes 29 produces or breaks up large quantities of optimum diameter droplets of the liquid form volatile anesthetic agent into the breathing air and which droplets evaporate into the breathing aire well before entering the patient.

The drop size can be adjusted by adjusting the diameter of the holes 29, the length of the holes and the shape of the holes in the first member, but also by adjusting the amplitude of the vibration, the mode of the vibration and the frequency of the vibration. The physical properties of the liquid anesthetics affect to the drop diameter also. The drop diameter increases proportionally as the diameter of the holes 29 in the first member 27 is increased whereas the length of the holes 29, the shape of the holes 29 as well as the change in the amplitude, in the vibration mode and/or in the frequency of the vibration may increase or decrease the drop diameter depending on the combination of all of those five parameters. For example if the frequency of the vibration is increased the drop diameter produced during one vibration may increase proportionally with the frequency up to some frequency, but above that frequency two or more smaller drops are produced during one vibration. Similar effect may be seen as the length of the holes 29 is increased as well as the shape of the holes 29 in to the direction perpendicular to the surface of vibrating membrane is changed from for example straight to conical or the amplitude of the vibration is increased or the mode of the vibration, the signal operating the vibrator 35, is changed for example from sinusoidal to triangular or square wave. The resulting effect is even more difficult to predict as all of these parameters above are changed at the same time.

The number of drops produced during each vibration depends on the number of the holes 29 in the first member 27 that may be 1000 holes or more, but preferably 10000 holes or more, but also the length of the holes 29 that may be 500 µm or less, but preferably less than 100 µm, the shape of the holes 29 that may be conical or other shape holes, but preferably staright holes, the amplitude of the vibration which may be tens of µm, but preferably more than 100 µm, the mode of the vibration which may be sinusoidal or similar, but preferably square wave type and the frequency of the vibration, which may be between 1-1000kHz, but preferably between 10-200 kHz. The first member 27 can contain thousands of holes 29, depending on the size of the first member 27, each hole 29 producing one or several drops during each vibration. Each hole 29 in the first member 27 is comparable to for example one pump head in the micro pump, thus the volume of the anesthetic agent turned in to drops with the vibrating first member 27 is enormous compared to the micro pump.

As an example optimum diameter of droplets is 1-5 µm that can be produced with 50 µm thick vibrating membrane containing a matrix of 7000 straight holes each having diameter of 5 µm. The total volume of sprayed anesthetic, as well as the droplet diameter depended on the liquid anesthetic sprayed, but the volume was between 0.1-1 ml/s, which is well enough for an adequate induction at the beginning of the sedation as well as for the stable state later on and the droplets evaporated into the breathing air before entering the intubation tube thus well before supplying the evaporated anesthetic agent mixed with the breathing air for the subject breathing. The square wave mode of vibration produced slightly better result compared to sinusoidal mode at the frequency of approximately 100 kHz, but also other resonant frequencies of vibrating membrane were possible. The increase in amplitude increased the volume of sprayed anesthetics.

In general the liquid form volatile anesthetic agent sprayed as small droplets of a diameter between 0.1 - 100 µm, but preferably between 1-10 µm, evaporates well before entering the patient without needing additional hardware. The vibrating first member 27 technique also ensures sufficient delivery efficiency during the induction also although the optimum drop diameter may vary slightly between different anesthetic liquids. The frequency of the vibration may vary between 1-1000 kHz, but is preferably between 10-200 kHz. The optimum diameter droplets are produced preferably with straight holes but can be produced with conical, tapered or other shape holes as well.

This invention discloses the optimum droplet diameter for the liquid form anesthetic agent that guarantee rapid passive vaporization of the agent without any additional hardware such as a heater element well before reaching the patient's lungs. The correct droplet diameter for the anesthetic agent is essential if no additional hardware is used. The correct droplet diameter, which is approximately less than 10um, but preferably 1-10 µm, guarantees that the liquid anesthetic evaporates before entering the patient lungs, but also guarantees sufficient delivery efficiency during the sedation and during the induction, too.

The written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. An apparatus for administering an anesthetic agent for a subject breathing comprising:
a liquid reservoir (9) for an anesthetic agent; and
an anesthetic agent dosing unit (7) in fluid connection with said liquid reservoir (9) and which anesthetic agent dosing unit is connectable to an airway tube (4) for delivering breathing gases to a subject, said dosing unit including a first member (27) to produce anesthetic agent droplets having a diameter less than 100 µm.

2. The apparatus according to claim 1 wherein the diameter of anesthetic agent droplets being 0,1 -100 µm.

3. The apparatus according to claim 1 wherein the diameter of anesthetic agent droplets being 1 - 10 µm, but preferably 1 - 5 µm.

4. The apparatus according to claim 1 wherein a frequency of the vibration may vary between 1-1000 kHz.

5. The apparatus according to claim 1 wherein a frequency of the vibration may vary between 10-200 kHz.

6. The apparatus according to claim 1 wherein said dosing unit (7) including said first member (27) has a group of holes (29) and also including a vibrator (35) making said first member (27) to vibrate so that anesthetic agent is adapted to flow through said holes (29) towards said airway tube (4).

7. The apparatus according to claim 6 wherein a diameter of the hole (29) is between 100 nm and 100 µm and a length of the hole is less than 500 µm.

8. A system for administering an anesthetic agent for a subject breathing comprising:
an anesthetic agent dosing unit (7);
a gas analyzing apparatus (6);
a user interface (11); and
an anesthetic agent delivery controller (8) connected to said gas analyzing apparatus (6) for receiving measurement results and also connected to said user interface (11) for receiving a target anesthetic agent concentration and further connected to said anesthetic agent dosing unit (7) for controlling an anesthetic agent delivery based on received measurement results and the target anesthetic agent concentration,
wherein said anesthetic agent dosing unit (7) is adapted to deliver anesthetic agent droplets having a diameter less than 100 µm.

9. The system according to claim 8 further comprising an anesthetic agent adsorber unit (5) adsorbing the anesthetic agent in a breathing air exhaled and to release the adsorbed anesthetic agent to the breathing air inhaled and which anesthetic agent adsorber unit is adapted to be mounted on an airway tube (4) with said anesthetic agent dosing unit (7) and said gas analyzing apparatus (6).

10. The system according to claim 8 wherein said anesthetic agent dosing unit (7) including a first member (27) having a group of holes (29) and also including a vibrator (35) making said first member (27) to vibrate so that anesthetic agent is adapted to flow through said holes (29) towards an airway tube (4) to produce anesthetic agent droplets having a diameter less than 100 µm.

11. A method for administering an anesthetic agent for a subject breathing comprising:
delivering a breathing air including at least oxygen for the subject breathing;
delivering a liquid containing the anesthetic agent;
breaking up said liquid into droplets having a diameter less than 100 µm;
delivering the droplets into said breathing air;
evaporating the droplets into said breathing air; and
supplying the evaporated anesthetic agent mixed with the breathing air for the subject breathing.

12. The method according to claim 11 further comprising adsorbing the anesthetic agent.

13. The method according to claim 12 further comprising releasing the adsorbed anesthetic agent into the breathing air.

14. The method according to claim 11 or 13 further comprising:
feeding a target anesthetic agent concentration;
measuring a concentration of the anesthetic agent component in the breathing air; and
comparing the measured concentration with the fed target anesthetic agent concentration.

15. The method according to claim 14 further comprising choosing one of regulating or not regulating the delivery of the anesthetic agent into the breathing air based on said comparison to achieve the target anesthetic agent concentration
